# EUROPÄISCHE PATENTANMELDUNG

(11) **EP 0 049 538 A2**
(43) Veröffentlichungstag der Anmeldung: **14.04.1982**
(21) Anmeldenummer: 81109562.9
(22) Anmeldetag: 02.07.1980
(51) Int. Cl.: A61K 31/17, A61K 31/18, A61K 31/275, A61K 31/19, A61K 31/47

(54) **Verwendung von Thioharnstoffderivaten als Arzneimittel bei der Behandlung von Fettstoffwechselstörungen**

(30) Priorität: 14.07.1979 DE 2928485
(62) Teilanmeldung aus: 80103765.6
(71) Anmelder: BAYER AG, 51368 Leverkusen (DE)
(72) Erfinder: Kabbe, Hans-Joachim, Dr., D-5090 Leverkusen (DE); Klauke, Erich, Dr., D-5068 Odenthal (DE); Krause, Hans Peter, Dr., D-5600 Wuppertal 2 (DE); Mardin, Mithat, Dr., D-5600 Wuppertal 1 (DE); Sitt, Rüdiger, Dr., D-5600 Wuppertal 1 (DE)

(57) **Zusammenfassung**

Die vorliegende Erfindung betrifft die Verwendung von Thioharnstoffderivaten der allgemeinen Formel I 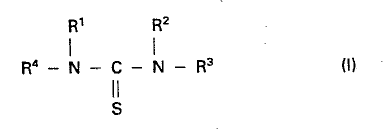 in welcher R¹, R², R³ und R⁴ die in der Beschreibung angegebene Bedeutung haben, zur Beeinflussung des Fettstoffwechsels sowie Arzneimittel enthaltend diese Verbindungen.

## Beschreibung

Die vorliegende Erfindung betrifft die Verwendung von Thioharnstoffderivaten zur Beeinflussung des Fettstoffwechsels sowie Arzneimittel enthaltend diese Verbindungen.

Einige der erfindungsgemäß verwendbaren Thioharnstoffderivate sind bereits bekannt (vergl. DE-OS 1 443 560; US-Pat. 3 335 142; US-Pat. 3 856 952; US-Pat. 3 903 130). Für diese bekannten Thioharnstoffderivate sind ebenfalls einige biologische Wirkungen beschrieben. Sie können z.B. als Herbizide, Bakterizide, Fungizide und Futterzusatzstoffe Verwendung finden. Ihre Wirkung auf den Fettstoffwechsel, insbesondere ihre lipidabsorptionshemmende Wirkung ist bisher noch nicht bekannt geworden.

Die vorliegende Erfindung betrifft die Verwendung von Thioharnstoffderivaten der allgemeinen Formel I

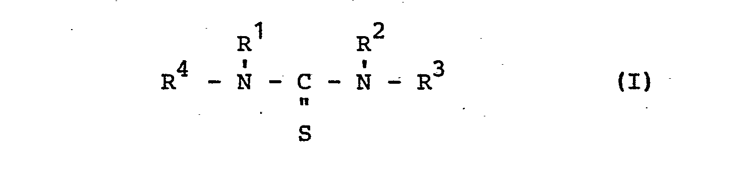

in welcher
_{R}¹ und _{R}² gleich oder verschieden sind und jeweils für Wasserstoff, geradkettiges, verzweigtes oder cyclisches Alkyl, für Aryl oder Aralkyl stehen, wobei die genannten Alkyl- und Arylreste ihrerseits gegebenenfalls substituiert sind durch Halogen oder Alkoxy,
_{R}³ und _{R}⁴ gleich oder verschieden sind und jeweils für einen Aryl- oder Heteroarylrest stehen, wobei diese Reste gegebenenfalls durch 1, 2, 3, oder 4 gleiche oder verschiedene Substituenten aus der Gruppe Nitro, Cyano, Halogen, Azido, Hydroxy, Amino, Carboxy, Aminocarbonyl, Aminosulfonyl, substituiert sind, wobei die Aminogruppen jeweils durch Alkyl oder Aryl 1₋ oder 2-fach substituiert sein können, Alkoxycarbonyl, Acyloxy, Acylamino, SO-Alkyl, S0₂-Alkyl, Acyl, Phenyl, Phenoxy, Phenylmercapto, Alkyl, Alkoxy oder Alkylmercapto, wobei die genannten Alkyl-, Alkoxy- und Alkylmercaptoreste ihrerseits gegebenenfalls durch ein oder mehrere Fluoratome substituiert sind und wobei die genannten Phenyl-, Phenylmercapto- und Phenoxysubstituenten ihrerseits wiederum substituiert sein können durch Halogen, Alkyl, Alkoxy oder Alkylmercapto, wobei die Alkyl-, Alkoxy- und Alkylmercapto-reste gegebenenfalls ein- oder mehrfach durch Fluor substituiert sind, oder wobei zwei benachbarte Substituenten am Arylrest gemeinsam mit den beiden Kohlenstoffatomen, an denen sie stehen, für einen gegebenenfalls durch Fluor substituierten Dioxan oder Dioxolring stehen.

bei der Behandlung von Erkrankungen des Fettstoffwechsels sowie bei der Herstellung von fettstoffwechselbeeinflussenden Arzneimitteln.

überraschenderweise zeigen die Thioharnstoffderivate der allgemeinen Formel I eine starke lipidabsorptionshemmende Wirkung. Bei Kenntnis des Standes der Technik konnte nicht erwartet werden, daß Verbindungen dieser Stoffklasse als lipidabsorptionshemmende Wirkstoffe verwendet werden können. Ihre bereits bekannte Verwendung als Futterzusatzstoffe in der Tierhaltung und ihre bakterizide Wirkung ließen erwarten, daß nach ihrer Applikation der Körper Nahrungsmittel verstärkt aufnimmt, was zu der in der Tierhaltung erwünschten Gewichtszunahme führt. Die lipidabsorptionshemmende Wirkung und die hieraus resultierende Möglichkeit, die Thioharnstoffderivate als Zusatzstoffe bei Nahrungsmitteln zu verwenden, bzw. durch Applikation entsprechender Arzneimittelformulierungen die Lipidabsorption aus Nahrungsmitteln zu hemmen, stellt die Überwindung eines aus dem Stand der Technik resultierenden Vorurteils dar.

Die Verwendung von Thioharnstoffderivaten bei der Behandlung von Hyperlipämien ermöglicht die Behandlung auch solcher Patienten, die gegenüber bereits bekannten Lipidabsorptionshemmern Unverträglichkeit oder Gewöhnun zeigen. Die erstmalige Verwendung der Thioharnstoffderivate als Wirkstoffe bei der Behandlung von Hyperlipämien stellt somit eine Bereicherung der Pharmazie dar.

Die Thioharnstoffderivate der allgemeinen Formel I werden in an sich bekannter Weise hergestellt, indem ma
a) ein Amin der allgemeinen Formel II 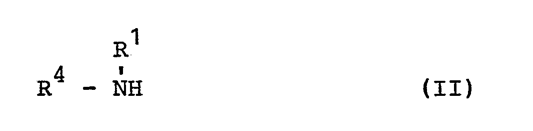 in welcher _{R}¹ und _{R}⁴ die oben angegebene Bedeutung haben, mit einer Verbindung der allgemeinen Formel III 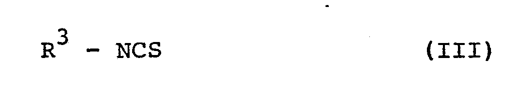 in welcher R³ die oben angegebene Bedeutung hat, in einem inerten organischen Lösungsmittel bei Temperaturen zwischen 20°C und 120°C umsetzt Variante a) ergibt symmetrische und unsymmetrische Harnstoffderivate, in denen R2 immer Wasserstoff bedeutet], oder
b) einen Thioester der allgemeinen Formel IV 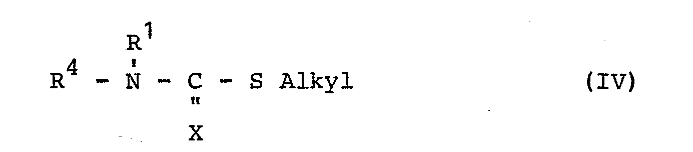 in welcher R und _{R}⁴ die oben angegebene Bedeutung haben, X für Schwefel oder Cyanamid steht, mit einem Amin der allgemeinen Formel V 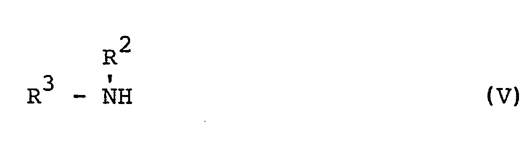 in welcher _{R}² und _{R}³ die oben angegebene Bedeutung haben,

gegebenenfalls in Gegenwart von inerten organischen Lösungsmitteln bei Temperaturen zwischen 20 und 180°C so lange umsetzt, bis die Alkylmercaptan-Entwicklung beendet ist, oder
c) eine Verbindung der allgemeinen Formel VI 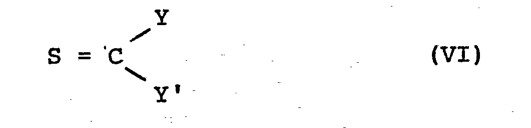 in welcher Y und Y' gleich oder verschieden sind und für einen nukleophil austauschbaren Rest wie Chlor, Alkylmercapto oder Phenoxy stehen, mit 2 Mol eines Amins der allgemeinen Formel (II) 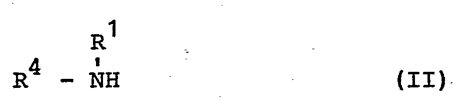 in welcher R¹ und R⁴ die oben angegebene Bedeutung haben, gegebenenfalls in Gegenwart von inerten organischen Lösungsmitteln umsetzt; [Variante c) ergibt immer symmetrische Harnstoffderivate, in denen jeweils _{R}¹ mit _{R}² und _{R}3 mit R⁴ identisch sind.]

Bei den vorstehenden Verfahrensvarianten a), b) und c) können die Amine der allgemeinen Formel (II) bzw. der allgemeinen Formel (V) jeweils alternativ eingesetzt werden, wenn die Bedeutung von _{R}¹ und _{R}⁴, bzw. von _{R}² und _{R}³ in dem jeweiligen Reaktionspartner vorgegeben ist.

Die Erfindung betrifft insbesondere die Verwendung von Thioharnstoffderivaten der allgemeinen Formel (I), in welcher
_{R}¹ und _{R}² gleich oder verschieden sind und jeweils für Wasserstoff, geradkettiges oder verzweigtes Alkyl mit 1 bis 4 Kohlenstoffatomen, Phenyl oder Benzyl stehen, wobei die genannten Alkyl- und Phenylreste ihrerseits gegebenenfalls substituiert sind durch Fluor oder Chlor,
_{R}³ und _{R}⁴ gleich oder verschieden sind und jeweils für einen Phenyl- oder Naphthylrest stehen, wobei diese Reste, insbesondere der Phenylrest, gegebenenfalls substituiert ist durch 1, 2, 3 oder 4 gleiche oder verschiedene Substituenten aus der Gruppe Nitro, Cyano, Halogen, insbesondere Fluor oder Chlor, Azido, Hydroxy, Amino, Carboxy, Aminocarbonyl, Aminosulfonyl, wobei die Aminogruppen jeweils durch Alkyl mit 1 bis 8 Kohlenstoffatomen oder Phenyl substituiert sein können, Phenyl, Phenoxy, Alkoxycarbonyl mit 1 bis 8 Kohlenstoffatomen, Acyloxy mit 1 bis 8 Kohlenstoffatomen, _{A}cylamido mit 1 bis 8 Kohlenstoffatomen, Acyl mit 1 bis 8 Kohlenstoffatomen, S0₂-Alkyl mit 1 bis 8 Kohlenstoffatomen, oder durch Alkyl, Alkoxy oder Alkylmercapto mit jeweils 1 bis 8 Kohlenstoffatomen, wobei diese Alkyl-, Alkoxy- und Alkylmercapto-Reste ihrerseits gegebenenfalls durch Fluor ein- oder mehrfach substituiert sind.
Vorzugsweise enthalten die vorgenannten Alkyl-, Alkoxy-, Alkylmercapto- und Acylreste 1 bis 4 Kohlenstoffatome.
Von besonderem Interesse ist die Verwendung von Verbindungen der allgemeinen Formel (I),
in welcher
_{R}¹ und _{R}² jeweils für Wasserstoff stehen,
_{R}³ und _{R}⁴ gleich oder verschieden sind und jeweils für Phenyl stehen, das durch Halogen, insbesondere Fluor oder Chlor, Trifluormethyl, Trifluormethoxy, Trifluormethylmercapto, Cyano, Carboxy, Alkyl, Alkoxy, Acyl, Alkoxycarbonyl oder Dialkylaminosulfonyl, mit jeweils 1 bis 4 Kohlenstoffatomen in den Alkyl-, Alkoxy- und Acylresten, ein-, zwei-, drei- oder vierfach substituiert ist.

Die Herstellung der neuen Verbindungen aus der durch die allgemeine Formel (I) definierten Stoffklasse erfolgt ebenfalls nach an sich bekannten Methoden gemäß den oben genannten Verfahrensvarianten a) und b)., wobei die als Ausgangsstoffe verwendeten Isocyanatderivate, Amine und Phenylcarbamidsäureester der allgemeinen Formel (III) und (IV) bekannt sind oder nach bekannten Methoden hergestellt werden können (vergl. R. Wagner et al, Synthetic Organic Chemistry, Wiley, New York, (1953), S. 640, 645, 653).

Die Thioharnstoffderivate gemäß Formel (I) zeigen eine vorteilhafte Hemmung der Lipidabsorption bei Mensch und Tier. Bei der Aufnahme fetthaltiger Nahrung führen sie zu einer geringeren alimentären Hyperlipamie, bei gleichzeitiger Hemmung der Cholesterinabsorption, so daß sie insbesondere zur Behandlung von Fettstoffwechselstörungen, wie z.B. Hyperlipoproteinämien, Atherosklerose oder Adipositas verwendet werden können.

Der Nachweis der vorteilhaften Wirkung läßt sich durch folgende Versuchsanordnung an Ratten zeigen:
Zur Erzeugung einer alimentären Hyperlipämie erhält eine Gruppe von Ratten 2,5 ml/kg Olivenöl per os verabreicht (Kontrollgruppe). Eine entsprechende Gruppe von anderen Ratten erhält gleichzeitig mit der Olivenölapplikation die Wirksubstanz als Suspension in Traganthschleim mit der Schludsonde verabreicht. Eine weitere Kontrollgruppe von Ratten erhält nur Traganthschleim appliziert.
2 Stunden nach der _{A}pplikatiön von Olivenöl werden die Konzentrationen der Serumtriglyceride in allen drei Ratten gruppen bestimmt (Methode: J. Ziegenhorst, Klin. Chem. 21, (1975) 1627). Zwei Stunden nach der Fettapplikation zeigen die nur mit Olivenöl behandelten Ratten (Gruppe 1) gegenüber den Ratten ohne Fettapplikation (Gruppe 3) einen deutlichen Anstieg der Serumtriglyceride. Mit diesem Anstieg, der gleich 100 % gesetzt wird, werden die verminderten Serumtriglycerid-Anstiege der mit Wirksubstanz und Olivenöl behandelten Tiere (Gruppe 2) verglichen. Es wurde gefunden, daß bereits geringe Dosierungen der Thioharnstoffderivate gemäß Formel (I) eine signifikante Senkung der Serumtriglyceride verursachen. Neben der starken lipidabsorptionshemmenden Wirkung zeigen die Verbindungen auch eine ausgesprochen gute Verträglichkeit.
Zur vorliegenden Erfindung gehören pharmazeutische Zubereitungen, die neben nichttoxischen, inerten pharmazeutisch geeigneten Trägerstoffen eine oder mehrere Verbindungen der obigen Formel enthalten oder die aus einer oder mehreren Verbindungen der obigen Formel bestehen sowie Verfahren zur Herstellung dieser Zubereitungen.
Zur vorliegenden Erfindung gehören auch pharmazeutische Zubereitungen in Dosierungseinheiten. Dies bedeutet, daß die Zubereitungen in Form einzelnen Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Lösungen, Suspensionen und Emulsionen und Pasten genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel., z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) - (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Lösungen und Emulsionen können neben dem oder den Wirkstoffen die üblichen Trägerstoffe, wie Lösungsmittel, Lösungsvermittler und Emulgatoren, z.B. Wasser, Aethylalkohol, Isopropylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Aethylalkohol, Propylenglykol, Suspendiermittel, z.B: äthoxylierte Isostearylalkohole, Polyoxy- äthylensorbit- und -sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl, und Süßmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gewichtsprozent der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört die Verwendung der Verbindungen der obigen Formel sowie die Verwendung von pharmazeutischen Zubereitungen, die eine oder mehrere Verbindungen der oben angegebenen Formel enthalten, in der Human- und Veterinärmedizin zur Verhütung, Besserung und/oder Heilung von Erkrankungen des Fettstoffwechsels.

Die Wirkstoffe oder die pharmazeutischen Zubereitungen können oral oder parenteral, vorzugsweise oral, appliziert werden.

Im allgemeinen hat es sich sowohl in der Human- als auch in der Veterinär-Medizin als vorteilhaft erwiesen, den oder die Wirkstoffe in Mengen von etwa 1,0 bis etwa 500, vorzugsweise 5 bis 100 mg/kg Körpergewicht je 24 Stunden, verteilt auf 1 bis 6 Verabreichungen, und zwar vor und/oder während und/oder nach der Mahlzeit zu applizieren. Eine Einzelgabe enthält den oder die Wirkstoffe vorzugsweise in MengenCvon etwa 1 bis etwa 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die folgenden Formulierungsbeispiele erläutern die Herstellung von erfindungsgemäß zu verwendenden Arzneimittelzubereitungen:

### Beispiele für Tablettenherstellung

1. 100 mg der Verbindung des Beispiels 1 werden mit 69 mg Milchzucker und 30 mg Maisstärke gemischt, anschließend mit einem Kleister aus 15 mg Maisstärke angeknetet und durch ein Sieb mit 3 - 5 mm Maschenweite gedrückt. Anschließend wird in einem Trockner bei 60 - 80°C getrocknet.
   Das erhaltene Granulat wird durch ein Sieb mit 0,8 mm Maschenweite geschlagen, weitere 15 mg Maisstärke, 10 mg Kalkum und 1 mg Magnesiumstearat werden zugemischt und mit Hilfe einer üblichen Tablettenpresse zu runden Tabletten mit 9 mm Durchmesser und einem Gesamtgewicht von 240 mg verpreßt.
2. 200 mg der Verbindung des Beispiels 29 werden mit 97 mg sekundären Calciumphosphat vermischt und mit einer wäßrigen Gelantinelösung, die 2 mg Gelantine enthält, angeknetet. Anschließend wird durch ein Sieb mit 3 - 5 mm Maschenweite gedrückt und bei 60 - 80°C getrocknet. Das trockene Granulat wird gesiebt (0,8 mm), anschließend 20 mg Weizenstärke und 1 mg Magnesiumstearat zugemischt und auf bekannte Weise tablettiert. Man erhält runde Tabletten vom Durchmesser 8 mm und einem Gesamtgewicht von 320 mg.

Besonders geeignet sind Thioharnstoffderivate der allgemeinen Formel (I), in der
_{R}¹ und R² für Wasserstoff stehen,
_{R}³ und _{R}⁴ jeweils für Phenyl stehen, welches durch 1, 2 oder 3 gleiche oder verschiedene Substituenten aus der Gruppe Chlor, Fluor, Cyano, Trifluormethyl, Trifluormethoxy, Trifluormethylmercapto, Dialkylaminosulphonyl, Alkoxycarbonyl oder Alkyl, mit jeweils 1 bis 4 Kohlenstoffatomen in den Alkyl- und Alkoxyresten, substituiert ist.

Die nachfolgenden Beispiele erläutern die Herstellung der erfindungsgemäß verwendbaren Wirkstoffe:

Bemerkung: In den nachfolgenden Beispielen besitzen die folgenden Abkürzungen folgende Bedeutung:
THF = Tetrahydrofuran
_{D}MSO = Dimethylsulfoxid
Tol = Toluol

### Beispiel 1 (Variante a)

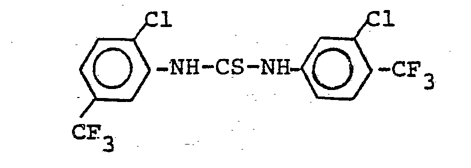

0,05 Mol 2-Chlor-5-trifluormethylanilin werden in 30 ml Tetrahydrofuran gelöst und mit einer Lösung von 0,05 Mol 3-Chlor-4-trifluormethylphenylisothiocyanat in 30 ml Tetrahydrofuran verrührt. Das Reaktionsgemisch wird auf 50°C erwärmt, wobei der N-2-Chlor-5-trifluormethylphenyl-N'-3-chlor-4-trifluormethylphenyl-thioharnstoff in einer Ausbeute von 82 % der Theorie ausfällt.

### Beispiel 2 (Variante b)

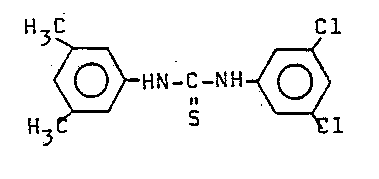

Eine Lösung von 0,2 Mol 3,5-Dichloranilin in 150 ml Tetrahydrofuran wird mit 30 ml Triethylamin versetzt. Man tropft 0,25 Mol Schwefelkohlenstoff zu, erhitzt 5 Stunden auf 50 - 60°C, kühlt auf 20°C ab und versetzt anschließend mit 0,25 Mol Methyljodid. Nach 2-stündigem Rühren bei 20 - 25°C werden 200 ml Xylol zugesetzt und die organische Phase mit Wasser ausgeschüttelt, anschließend eingeengt und mit 0,2 Mol 3,5-Dimethylanilin versetzt. Dann wird das Reaktionsgemisch bis zum Nachlassen der Methylmercaptanentwicklung auf 80 - 140°C erhitzt. Man erhält N-3,5-Dichlorphenyl-N'-3,5-dimethylphenyl-thioharnstoff, der nach dem Abkühlen abgesaugt wird.
Schmelzpunkt: 265 - 267°C; Ausbeute: 49 % der Theorie.

### Beispiel 3 (Variante a)

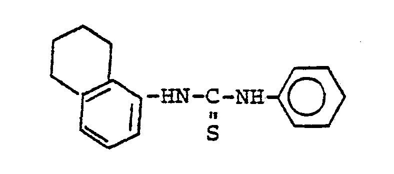

0,1 Mol 5-Aminotetralin werden in 30 ml Toluol gelöst und mit 0,1 Mol Phenylsenföl versetzt, wobei die Temperatur auf 35°C ansteigt und sich ein Niederschlag bildet. Man läßt 24 Stunden stehen und saugt den N-5-Tetralyl-N'-phenyl-thioharnstoff ab.
Schmelzpunkt: 145 - 147°C; Ausbeute: 83 % der Theorie.

Die folgenden Tabellenbeispiele werden, wenn nicht ausdrücklich anders angegeben, gemäß Variante a) analog Beispiel 1 hergestellt:

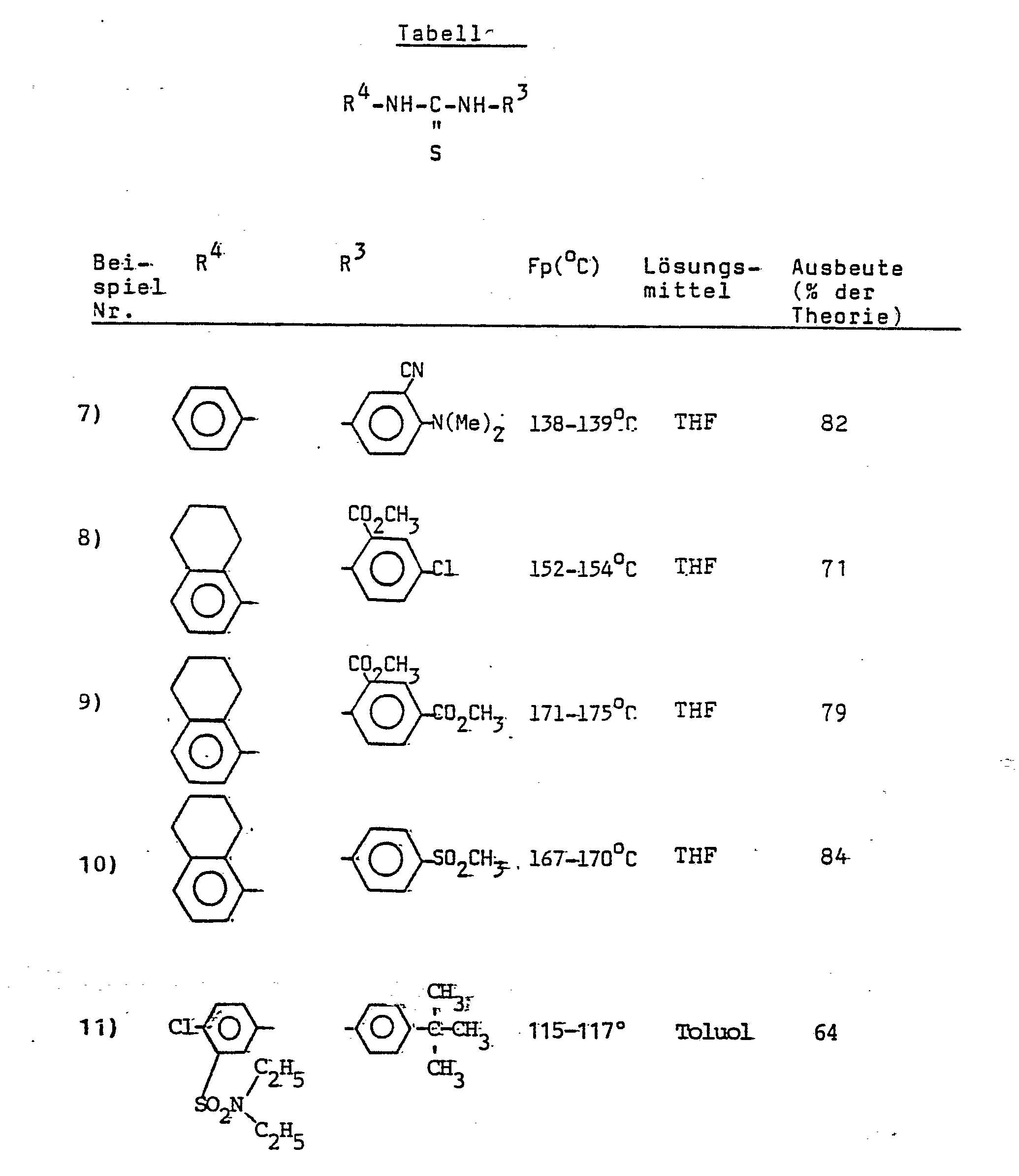

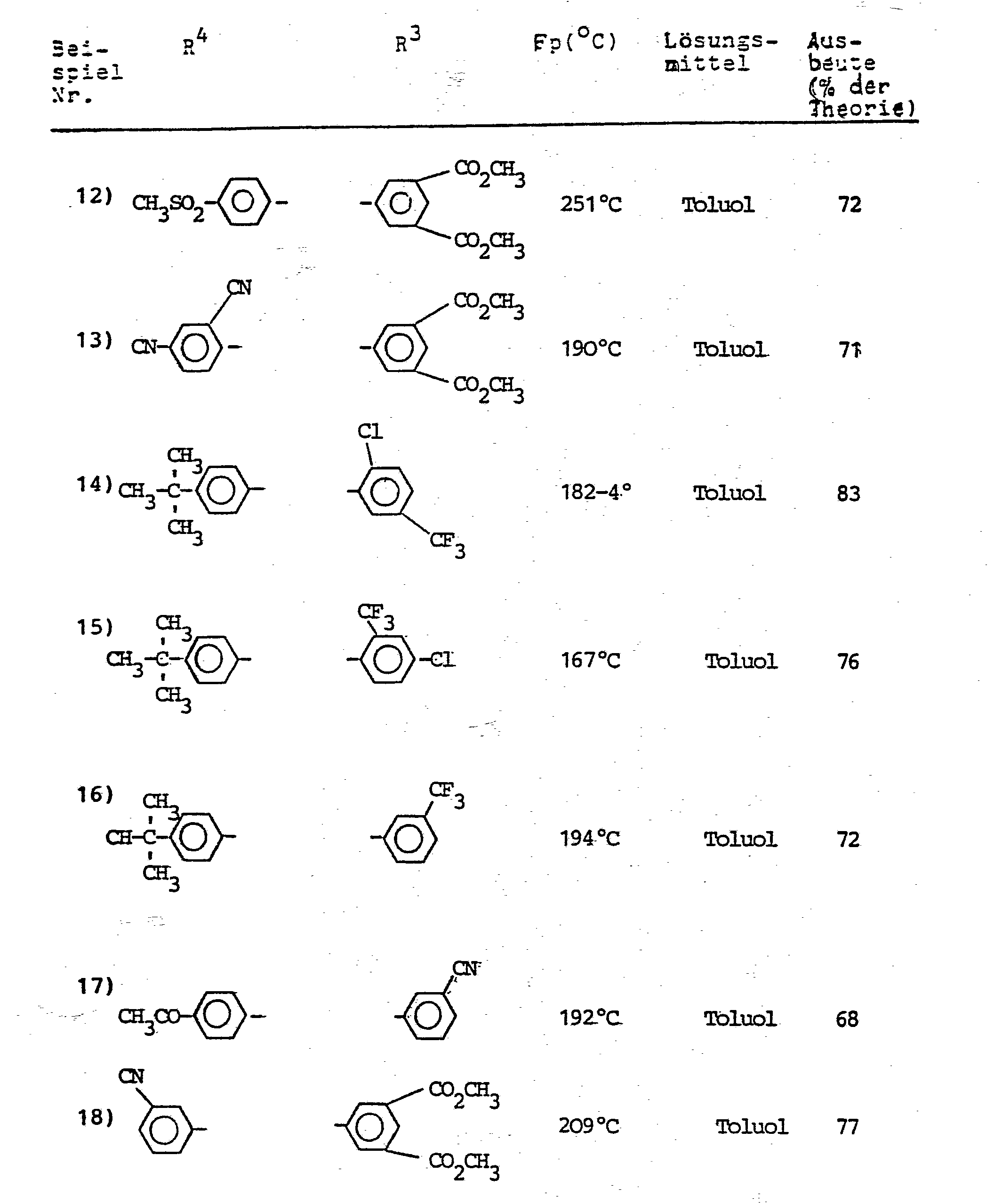

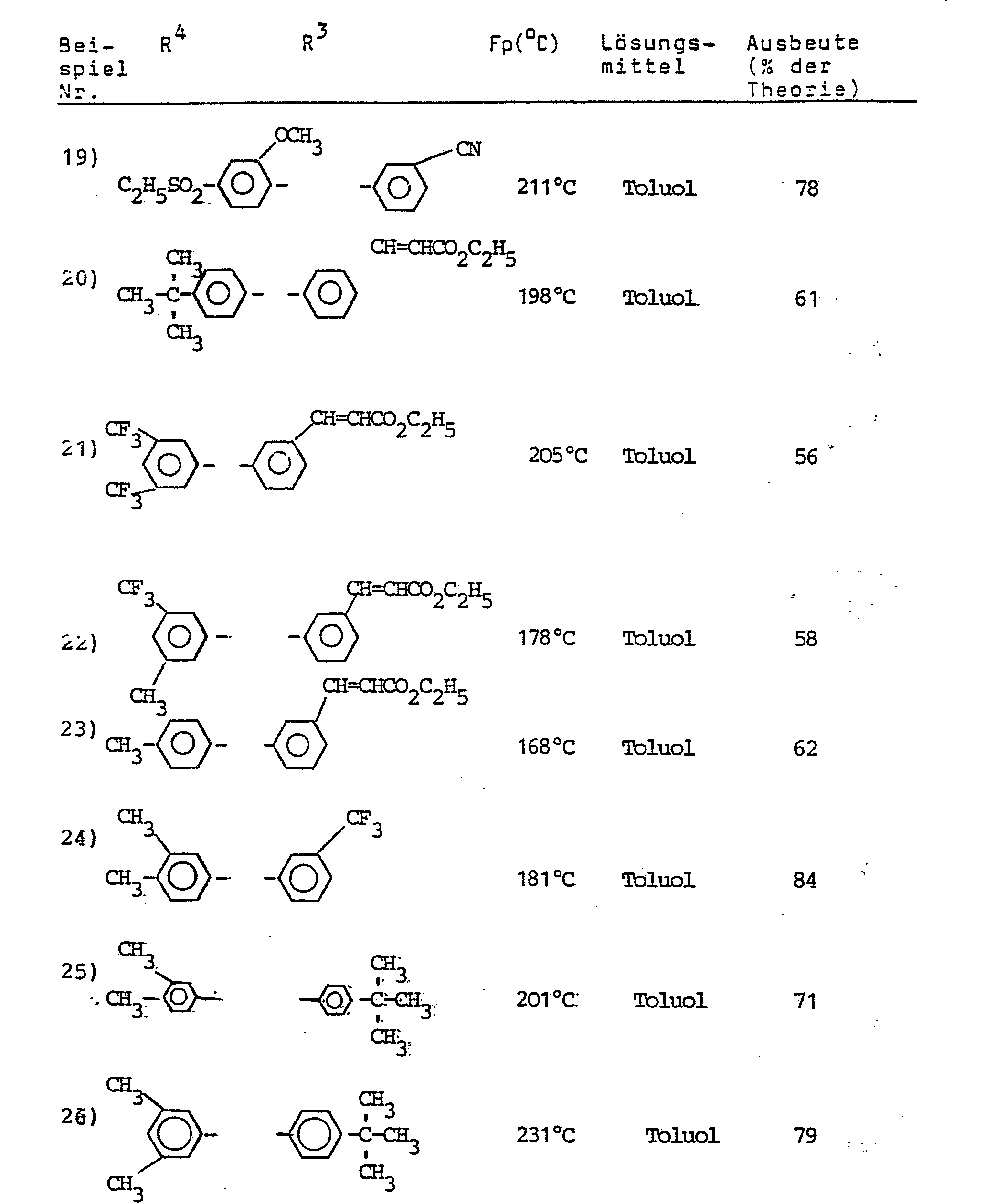

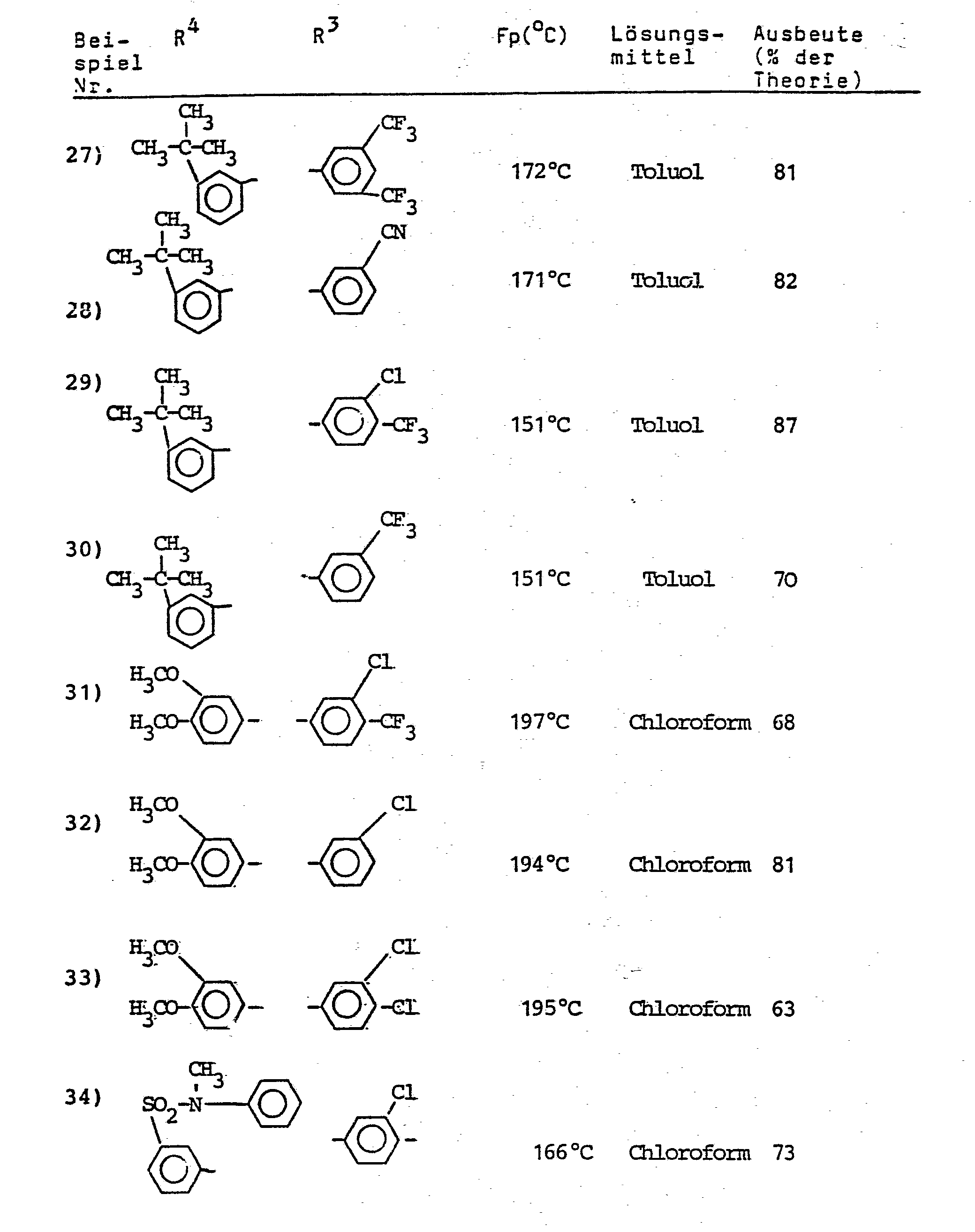

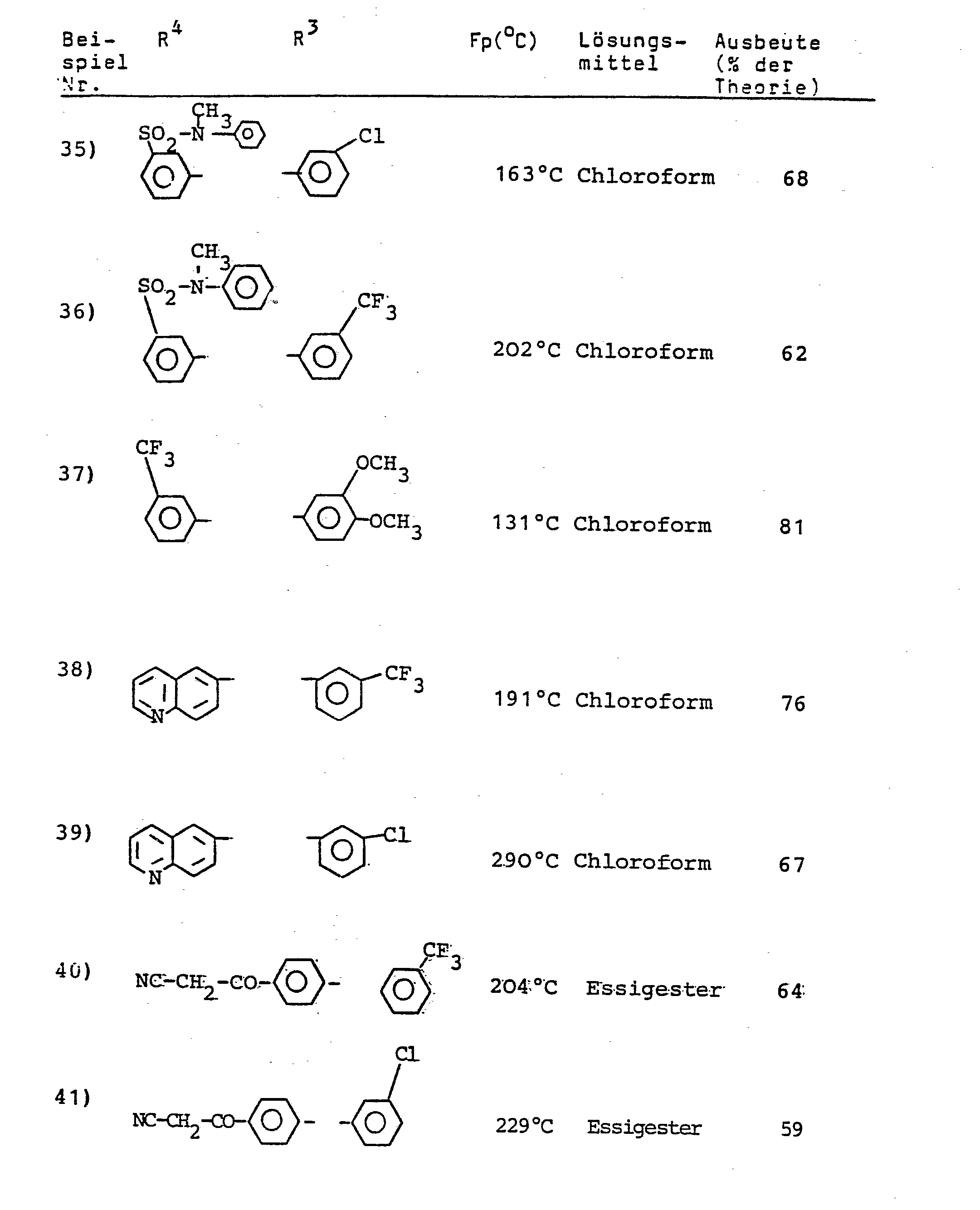

## Patentansprüche

1. Verwendung von Thioharnstoffderivaten der allgemeinen Formel I 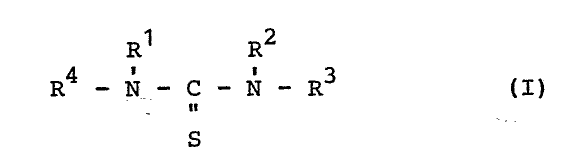 in welcher
R¹ und R² gleich oder verschieden sind und jeweils für Wasserstoff, geradkettiges, verzweigtes oder cyclisches Alkyl, für Aryl oder Aralkyl stehen, wobei die genannten Alkyl-und Arylreste ihrerseits gegebenenfalls substituiert sind durch Halogen oder Alkoxy,
_{R}³ und _{R}⁴ gleich oder verschieden sind und jeweils für einen Aryl- oder Heteroarylrest stehen, wobei diese Reste gegebenenfalls durch 1, 2, 3 oder 4 gleiche oder verschiedene Substituenten aus der Gruppe Nitro, Cyano, Halogen, Azido, Hydroxy, Amino, Carboxy, Aminocarbonyl, Aminosulfonyl substituiert sind, wobei die Aminogruppen jeweils durch Alkyl oder Aryl ein- oder zweifach substituiert sein können, Alkoxycarbonyl, Acyloxy, Acylamino, SO-Alkyl, S0₂-Alkyl, Acyl, Phenyl, Phenoxy, Phenylmercapto, Alkyl, Alkoxy oder Alkylmercapto, wobei die genannten Alkyl-, Alkoxy- und Alkylmercaptoreste ihrerseits gegebenenfalls durch ein oder mehrere Fluoratome substituiert sind und wobei die genannten Phenyl-, Phenylmercapto- und Phenoxysubstituenten ihrerseits wiederum substituiert sind, oder wobei zwei benachbarte Substituenten am Arylrest gemeinsam mit den beiden Kohlenstoffatomen, an denen sie stehen, für einen gegebenenfalls durch Fluor substituierten Dioxan oder Dioxolring stehen.
bei der Behandlung von Erkrankungen des Fettstoffwechsels.

2. Verwendung gemäß Anspruch 1 von Verbindungen der allgemeinen Formel I,
in welcher
_{R}¹ und _{R}² gleich oder verschieden sind und jeweils für Wasserstoff, geradkettiges oder verzweigtes Alkyl mit 1 bis 4 Kohlenstoffatomen, Phenyl oder Benzyl stehen, wobei die genannten Alkyl- und Phenylreste ihrerseits gegebenenfalls substituiert sind durch Fluor oder Chlor,
_{R}³ und _{R}⁴ gleich oder verschieden sind und jeweils für einen Phenyl- oder Naphthylrest stehen, wobei diese Reste, insbesondere der Phenylrest, gegebenenfalls substituiert ist durch 1, 2, 3 oder 4 gleiche oder verschiedene Substituenten aus der Gruppe Nitro, Cyano, Halogen, insbesondere Fluor oder Chlor, Azido, Hydroxy, Amino, Carboxy, Aminocarbonyl, Aminosulfonyl, wobei die Aminogruppen jeweils durch Alkyl mit 1 bis 8 Kohlenstoffatomen oder Phenyl substituiert sein können, Phenyl, Phenoxy, Alkoxycarbonyl mit 1 bis 8 Kohlenstoffatomen, Acyloxy mit 1 bis 8 Kohlenstoffatomen, Acylamido mit 1 bis 8 Kohlenstoffatomen, Acyl mit 1 bis 8 Kohlenstoffatomen, oder durch Alkyl, Alkoxy oder Alkylmercapto jeweils 1 bis 8 Kohlenstoffatomen, wobei diese Alkyl-, _{A}lkoxy- und Alkylmercapto-Reste ihrerseits gegebenenfalls durch Fluor ein- oder mehrfach substituiert sind.

3. Verwendung gemäß Anspruch 1 von Verbindungen der allgemeinen Formel I,
in welcher
_{R}¹ und _{R}² jeweils für Wasserstoff stehen,
R³ und R⁴ gleich oder verschieden sind und jeweils für Phenyl stehen, das durch Halogen, insbesondere Fluor oder Chlor, Trifluormethyl, Trifluormethoxy, Trifluormethylmercapto, Cyano, Carboxy, Alkyl, Alkoxy, Acyl, Alkoxycarbonyl oder Dialkylaminosulfonyl, mit jeweils 1 bis 4 Kohlenstoffatomen in den Alkyl-, Alkoxy- und Acylresten, ein-, zwei-, drei- oder vierfach substituiert ist.

4. Verwendung gemäß Anspruch 1 von Verbindungen der allgemeinen Formel I,
in welcher
_{R}¹ und _{R}² für Wasserstoff,
_{R}³ und _{R}⁴ jeweils für Phenyl stehen, welches durch 1, 2 oder 3 gleiche oder verschiedene Substituenten aus der Gruppe Chlor, Fluor, Cyano, Trifluormethyl, Trifluormethoxy, Trifluormethylmercapto, Dialkylaminosulphonyl, Alkoxycarbonyl oder Alkyl, mit jeweils 1 bis 4 Kohlenstoffatomen in den Alkyl-und Alkoxyresten, substituiert ist.

5. Lipidabsorptionshemmende Arzneimittel enthaltend mindestens eine Verbindung der allgemeinen Formel I gemäß Anspruch 1.

6. Verfahren zur Herstellung von lipidabsorptionshemmenden Arzneimitteln, dadurch gekennzeichnet, daß man mindestens eine Verbindung der allgemeinen Formel I gemäß Anspruch 1 mit üblichen Hilfs- und Trägerstoffen in eine geeignete Applikationsform überführt.
